# DEMANDE DE BREVET EUROPEEN

(11) **EP 2 345 402 A1**
(43) Date de publication de la demande: **20.07.2011**
(21) Numéro de dépôt: 10194666.3
(22) Date de dépôt: 13.12.2010
(51) Int. Cl.: A61K 8/365, A61Q 19/10, A61K 8/04

(54) **Composition cosmétique de nettoyage comprenant un dérivé d'acide jasmonique et un tensioactif**

(30) Priorité: 22.12.2009 FR 0959371
(71) Demandeur: L'Oréal, 75008 Paris (FR)
(72) Inventeur: Bernard, Anne-Laure, 92160, Antony (FR)
(74) Mandataire: Duvert, Sandra

(57) **Abrégé**

L'invention se rapporte à une composition cosmétique de nettoyage comprenant, (1) au moins un tensioactif moussant anionique ou amphotère et (2) au moins un composé dérivé d'acide jasmonique choisi parmi ceux répondant à la formule (I) suivante : 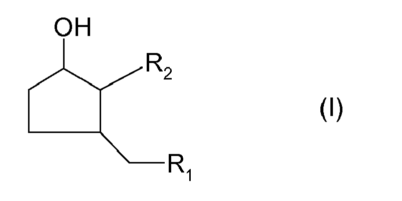 dans laquelle :
R1 représente un radical COOR3, R3 désignant un atome d'hydrogène ou un radical alkyle en C1-C4, éventuellement substitué par un ou plusieurs groupes hydroxyle ;
R2 représente un radical hydrocarboné, saturé ou insaturé, linéaire ayant de 1 à 18 atomes de carbones, ou ramifié ou cyclique ayant de 3 à 18 atomes de carbone ;

ainsi que leurs isomères optiques, et sels correspondants.

## Description

L'invention se rapporte à une composition cosmétique de nettoyage contenant dans un milieu aqueux, un dérivé de l'acide jasmonique et un tensioactif moussant anionique ou amphotère, et à ses utilisations pour le nettoyage et/ou le démaquillage des matières kératiniques telles que la peau, les muqueuses, les lèvres, et/ou les fibres kératiniques telles que les cils ou les cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons. Un moyen pour bien nettoyer la peau est d'utiliser des produits de nettoyage moussants.

Une des difficultés majeures de la formulation de produits moussants consiste à mettre au point des produits ayant un très bon pouvoir moussant , les performances de mousse étant en général reliées par les consommatrices à un nettoyage efficace. Pour la formulation d'un produit d'une telle qualité, il est nécessaire d'utiliser des tensioactifs moussants pour assurer une mousse rapide, abondante, dense (qui ne coule pas sur le visage), facile à étaler et qui permettra un bon rinçage,

La demanderesse a découvert de manière surprenante que l'utilisation d'un dérivé d'acide jasmonique particulier en association avec un tensioactif moussant anionique ou amphotère permet d'améliorer les qualités de la mousse des compositions nettoyantes.

Ainsi, l'invention a pour objet une composition cosmétique de nettoyage contenant, (1) au moins un tensioactif moussant anionique ou amphotère et (2) au moins un composé dérivé d'acide jasmonique choisi parmi ceux répondant à la formule (I) suivante : 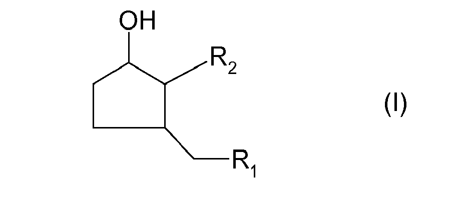 dans laquelle :
- R1 représente un radical COOR3, R3 désignant un atome d'hydrogène ou un radical alkyle en C1-C4, éventuellement substitué par un ou plusieurs groupes hydroxyle ;
- R2 représente un radical hydrocarboné, saturé ou insaturé, linéaire ayant de 1 à 18 atomes de carbones, ou ramifié ou cyclique ayant de 3 à 18 atomes de carbone ;
ainsi que leurs isomères optiques, et sels correspondants.

La composition de l'invention présente un bon pouvoir moussant, notamment une mousse dense et abondante, tout en étant bien rinçable.

La composition de l'invention étant une composition cosmétique, elle contient un milieu physiologiquement acceptable. On entend ici par « milieu physiologiquement acceptable », un milieu compatible avec les matières kératiniques. Il s'agit de préférence d'un milieu cosmétiquement acceptable, c'est-à-dire qui, en outre, présente une couleur, une odeur et un toucher agréables et qui ne génère pas d'inconforts inacceptables (picotements, tiraillements, rougeurs), susceptibles de détourner la consommatrice d'utiliser cette composition.

La composition de nettoyage de l'invention est une composition moussante qui est rincée après application sur la peau.

### Dérivé d'acide jasmonique

Le composé dérivé d'acide jasmonique est un composé choisi parmi ceux répondant à la formule (I) suivante : 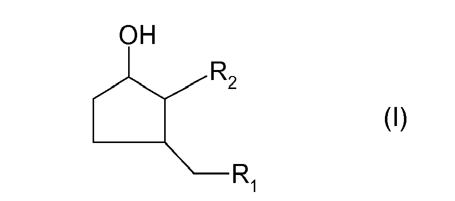 dans laquelle :
R1 représente un radical COOR3, R3 désignant un atome d'hydrogène ou un radical alkyle en C1-C4 , éventuellement substitué par un ou plusieurs groupes hydroxyle ;
R2 représente un radical hydrocarboné, saturé ou insaturé, linéaire ayant de 1 à 18 atomes de carbones, ou ramifié ou cyclique ayant de 3 à 18 atomes de carbone ;
ainsi que leurs isomères optiques, et sels correspondants.

De préférence, R1 désigne un radical choisi parmi ―COOH, -COOMe, -COO-CH2-CH3, - COO—CH2-CH(OH)-CH2OH, -COOCH2-CH2-CH2OH , -COOCH2-CH(OH)-CH3 . Préférentiellement, R1 désigne un radical ―COOH.

Préférentiellement, R2 désigne un radical hydrocarboné, linéaire, saturé ou insaturé, et de préférence ayant de 2 à 7 atomes de carbone. En particulier, R₂ peut être un radical pentyl, pentenyl, hexyle, heptyle.

Selon un mode de réalisation, le composé de formule (I) est choisi parmi l'acide 3-hydroxy-2-[(2Z)-2-pentenyl]-cyclopentane acétique ou l'acide 3-hydroxy 2-pentyl cyclopentane acétique, et de préférence est l'acide 3-hydroxy 2-pentyl cyclopentane acétique.

Les sels des composés utilisables selon l'invention sont en particulier choisis parmi les sels de métal alcalin, par exemple sodium, potassium ; les sels de métal alcalino-terreux, par exemple calcium, magnésium, strontium, les sels métalliques, par exemple zinc, aluminium, manganèse, cuivre ; les sels d'ammonium de formule NH₄⁺ ; les sels d'ammonium quaternaires ; les sels d'amines organiques, comme par exemple les sels de méthylamine, de diméthylamine, de triméthylamine, de triéthylamine, d'éthylamine, de 2-hydroxyéthylamine, de bis-(2-hydroxyéthyl)amine, de la tri-(2-hydroxyéthyl)amine ; les sels de lysine, d'arginine. On utilise de préférence les sels choisis parmi les de sodium, potassium, calcium , magnésium, strontium, cuivre, manganèse, zinc.

Le composé dérivé d'acide jasmonique de formule (I) peut être présent en une quantité allant de 0,1 à 15 % en poids, de préférence de 0,2 à 10% en poids et mieux de 0,5 à 5% en poids par rapport au poids total de la composition.

### Tensioactifs anioniques et amphotères

Les tensioactifs anioniques et amphotères (ou zwitterioniques) présents dans la composition selon l'invention sont de préférence des tensioactifs moussants aptes à nettoyer la peau.

Les tensioactifs moussants sont des détergents et se différencient des émulsionnants par la valeur de leur HLB (Hydrophilic Lipophilic balance), le HLB étant le rapport entre la partie hydrophile et la partie lipophile dans la molécule. Le terme HLB est bien connu de l'homme du métier et est décrit par exemple dans "The HLB system. A time-saving guide to Emulsifier Selection" (published by ICI Americas Inc ; 1984). Pour les émulsionnants, le HLB va généralement de 3 à 8 pour la préparation des émulsions E/H et de 8 à 18 pour la préparation des émulsions H/E, alors que les tensioactifs moussants ont généralement un HLB supérieur à 20.

Les tensioactifs moussants anioniques et/ou amphotères peuvent être présents dans la composition en une quantité (en matière active) allant par exemple de 0,1 à 40 % en poids, de préférence de 0,5 à 30 % en poids, mieux de 1 à 25% et encore mieux de 3 à 20% en poids par rapport au poids total de la composition.

### Tensioactifs anioniques

Les tensioactifs anioniques pouvant être ajoutés dans la composition selon l'invention peuvent être choisis notamment parmi les dérivés anioniques de protéines d'origine végétale ou de protéines de soie, les phosphates et alkylphosphates, les carboxylates, les sulfosuccinates, les dérivés des aminoacides, les alkyl sulfates, les alkyl éther sulfates, les sulfonates, les iséthionates, les taurates, les alkyl sulfoacétates, les polypeptides, les dérivés anioniques d'alkyl polyglucoside, les savons (sels d'acides gras) et leurs mélanges.
a) Les dérivés anioniques de protéines d'origine végétale sont des hydrolysats de protéine à groupement hydrophobe, ledit groupement hydrophobe pouvant être naturellement présent dans la protéine ou être ajouté par réaction de la protéine et/ou de l'hydrolysat de protéine avec un composé hydrophobe. Les protéines sont d'origine végétale ou dérivées de soie, et le groupement hydrophobe peut être notamment une chaîne grasse, par exemple une chaîne alkyle comportant de 10 à 22 atomes de carbone. Comme dérivés anioniques de protéines d'origine végétale, on peut plus particulièrement citer les hydrolysats de protéines de pomme, de blé, de soja, d'avoine, comportant une chaîne alkyle ayant de 10 à 22 atomes de carbone et leurs sels. La chaîne alkyle peut être notamment une chaîne lauryle et le sel peut être un sel de sodium, de potassium et/ou d'ammonium.
   Ainsi, comme hydrolysats de protéine à groupement hydrophobe, on peut citer par exemple les sels des hydrolysats de protéine de soie modifiée par l'acide laurique, tels que le produit commercialisé sous la dénomination KAWA SILK par la société Kawaken ; les sels des hydrolysats de protéine de blé modifiée par l'acide laurique, tels que le sel de potassium commercialisé sous la dénomination Aminofoam W OR par la société Croda (nom CTFA : Potassium lauroyl wheat aminoacids) et le sel de sodium commercialisé sous la dénomination PROTEOL LW 30 par la société Seppic (nom CTFA : sodium lauroyl wheat aminoacids) ; les sels des hydrolysats de protéine d'avoine comportant une chaîne alkyle ayant de 10 à 22 atomes de carbone, et plus spécialement les sels des hydrolysats de protéine d'avoine modifiée par l'acide laurique, tels que le sel de sodium commercialisé sous la dénomination PROTEOL OAT (soltion aqueuse à 30%) par la société Seppic (nom CTFA : Sodium lauroyl oat aminoacids) ; les sels des hydrolysats de protéine de pomme, comportant une chaîne alkyle ayant de 10 à 22 atomes de carbone, tels que le sel de sodium commercialisé sous la dénomination PROTEOL APL (solution hydroglycolique à 30%) par la société Seppic (nom CTFA : Sodium Cocoyl Apple amino acids). On peut citer aussi le mélange de lauroyl-aminoacides (aspartique, glutamique, glycine, alanine) neutralisé au N-methylglycinate de sodium, commercialisé sous la dénomination PROTEOL SAV 50 S par la société Seppic (nom CTFA : Sodium Cocoyl amino acids).
b) Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de monoalkyl (C12-C13) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie, et le cétylphosphate de potassium commercialisé sous la dénomination ARLATONE MAP 160K par la société Uniqema.
c) Comme carboxylates, on peut citer :
   ● Les amido éthercarboxylates (AEC), comme le Lauryl amido ether carboxylate de sodium (3 OE), commercialisé sous la dénomination AKYPO FOAM 30® par la société Kao Chemicals.
   ● les sels d'acides carboxyliques polyoxyéthylénés, comme le Lauryl ether carboxylate de sodium (C₁₂₋₁₄₋₁₆ 65/25/10) oxyéthyléné (6 OE) commercialisé sous la dénomination AKYPO SOFT 45 NV® par la société Kao Chemicals, les acides gras d'origine huile d'olive polyoxyéthylénés et carboxyméthylés commercialisés sous la dénomination OLIVEM 400® par la société BIOLOGIA E TECNOLOGIA, le tri-decyl ether carboxylate de sodium oxyéthyléné (6 OE) commercialisé sous la dénomination NIKKOL ECTD-6NEX ® par la société Nikkol.
   ● les sels d'acides gras (savons) ayant une chaîne alkyl en C6 à C22, neutralisés par une base organique ou minérale telle que la potasse, la soude, la triéthanolamine,la N-methyl glucamine, la lysine et l'arginine.
d) Comme dérivés des aminoacides, on peut citer notamment les sels alcalins d'aminoacides, tels que :
   ● les sarcosinates, comme le Lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium commercialisé sous la dénomination NIKKOL SARCOSINATE MN® par la société Nikkol, le palmitoyl sarcosinate de sodium commercialisé sous la dénomination NIKKOL SARCOSINATE PN ® par la société Nikkol.
   ● les alaninates, comme le N-lauroyl-N methyl amidopropionate de sodium commercialisé sous la dénomination SODIUM NIKKOL ALANINATE LN 30® par la société Nikkol, ou commercialisé sous la dénomination ALANONE ALE® par la société Kawaken, le N-lauroyl N-methyl alanine triéthanolamine commercialisé sous la dénomination ALANONE ALTA ® par la société Kawaken.
   ● Les glutamates, comme le mono-cocoyl glutamate de triéthanolamine commercialisé sous la dénomination ACYLGLUTAMATE CT-12® par la société Ajinomoto, le lauroylglutamate de triéthanolamine commercialisé sous la dénomination ACYLGLUTAMATE LT-12® par la société Ajinomoto.
   ● les aspartates, comme le mélange de N-lauroyl aspartate de triethanolamine /N-myristoyl aspartate de triethanolamine commercialisé sous la dénomination ASPARACK® par la société Mitsubishi.
   ● les dérivés de glycine (glycinates), comme le N-cocoyl glycinate de sodium commercialisé sous les dénominations AMILITE GCS-12® et AMILITE GCK 12 par la société Ajinomoto.
   ● Les citrates tels que le mono-ester citrique d'alcools de coco oxyéthylénés (9 moles), commercialisé sous la dénomination WITCONOL EC 1129 par la société Goldschmidt.
   ● les galacturonates tels que le dodeécyl d-galactoside uronate de sodium commercialisé par la société Soliance.
e) Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco. On peut utiliser aussi les sulfosuccinates de polydimethylsiloxane tels que le disodium PEG-12 dimethicone sulfosuccinate commercialisé sous la dénomination MACKANATE-DC30 par la société Mac Intyre.
f) Comme alkyl sulfates, on peut citer par exemple le lauryl sulfate de triéthanolamine (nom CTFA : TEA-lauryl sulfate) tel que le produit commercialisé par la société Huntsman sous la dénomination EMPICOL TL40 FL ou celui commercialisé par la société Cognis sur la dénomination TEXAPON T42, produits qui sont à 40% en solution aqueuse. On peut aussi citer le lauryl sulfate d'ammonium (nom CFTA : Ammonium lauryl sulfate) tel que le produit commercialisé par la société Huntsman sous la dénomination EMPICOL AL 30FL qui est à 30% en solution aqueuse.
g) Comme alkyl éther sulfates, on peut citer par exemple le lauryl éther sulfate de sodium (nom CTFA : sodium laureth sulfate) comme celui commercialisé sous les dénominations TEXAPON N40 et TEXAPON AOS 225 UP par la société Cognis, le lauryl éther sulfate d'ammonium (nom CTFA : ammonium laureth sulfate) comme celui commercialisé sous la dénomination STANDAPOL EA-2 par la société Cognis.
h) Comme sulfonates, on peut citer par exemple les alpha-oléfines sulfonates comme l'alpha-oléfine sulfonate de sodium (C14-16) commercialisé sous la dénomination BIO-TERGE AS-40® par la société Stepan, commercialisé sous les dénominations WITCONATE AOS PROTEGE® et SULFRAMINE AOS PH 12® par la société Witco ou commercialisé sous la dénomination BIO-TERGE AS-40 CG® par la société Stepan, l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40®, MANROSOL SXS93® par la société Manro.
i) Comme iséthionates, on peut citer les acyliséthionates comme le cocoyl-iséthionate de sodium, tel que le produit commercialisé sous la dénomination JORDAPON Cl P® par la société Jordan.
j) Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.
k) Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia, le sel di-sodique d'ester sulfosuccinique de cocoylpolyglucoside (1,4), commercialisé sous la dénomination ESSAI 512 MP® par la société Seppic, le sel de sodium d'ester citrique de cocoyl polyglucoside (1,4) commercialisé sous la dénomination EUCAROL AGE-EC® par la société Cesalpinia.
l) Les savons sont obtenus à partir d'un acide gras qui est partiellement ou totalement saponifié (neutralisé) par un agent basique. Ce sont des savons de métal alcalin ou alcalino-terreux ou de bases organiques. Comme acides gras, on peut utiliser les acides gras saturés, linéaires ou ramifiés, comportant de 8 à 30 atomes de carbone, et de préférence comportant de 8 à 22 atomes de carbone. Cet acide gras peut être en particulier choisi parmi l'acide palmitique, l'acide stéarique, l'acide myristique, l'acide laurique et leurs mélanges.
   Comme agents basiques, on peut utiliser par exemple les hydroxydes de métaux alcalins (hydroxyde de sodium et hydroxyde de potassium ou potasse), les hydroxydes de métaux alcalino-terreux (par exemple de magnésium), l'hydroxyde d'ammonium, ou encore les bases organiques comme la triéthanolamine, la N-méthylglucamine, la lysine et l'arginine.
   Les savons peuvent être notamment des sels alcalins d'acide gras, l'agent basique étant un hydroxyde de métal alcalin, et de préférence l'hydroxyde de potassium ou potasse (KOH).
   La quantité d'agent basique doit être suffisante pour que l'acide gras soit au moins partiellement neutralisé.

De préférence le tensioactif anionique est choisi parmi les alkyl sulfates, les alkyl éther sulfates tel que le lauryl éther sulfate de sodium, les iséthionates, les dérivés d'aminoacides, en particulier les dérivés de glycine (glycinates), comme le N-cocoyl glycinate de sodium et leurs mélanges.

### Tensioactifs moussants amphotères et zwitterioniques

Les tensioactifs amphotères et zwitterioniques peuvent être choisis par exemple parmi les bétaïnes, les N-alkylamidobétaïnes et leurs dérivés, les sultaïnes, les alkyl polyaminocarboxylates, les alkylamphoacétates et leurs mélanges.

Comme bétaïnes, on peut citer notamment les alkylbétaïnes comme par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER (10 OE) BETAINE® par la société Shin Nihon Rica, la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou commercialisée sous la dénomination EMPIGEN BB® par la société Albright & Wilson, la lauramidopropyl bétaïne commercialisée sous la dénomination REWOTERIC AMB12P® par la société Witco.

Comme sultaines, on peut citer les hydroxylsultaïnes, telles que la Cocamidopropyl hydroxysultaïne comme le produit commercialisé sous la dénomination REWOTERIC AM CAS par la société Golschmidt-Degussa, ou le produit commercialisé sous la dénomination CROSULTAINE C-50® par la société Croda.

Come alkyl polyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA: disodium cocoamphodiacetate) comme le produit commercialisé sous la dénomination MIRANOL C2M CONCENTRE NP® par la société Rhodia, le N-cocoyl-N-hydroxyéthyl-N-carboxyméthyl-éthylènediamine N-sodique (nom CTFA : sodium cocamphoacetate), le cocoamphohydroxypropyl sulfonate de sodium commercialisé sous la dénomination MIRANOL CSE par la société Rhodia.

De préférence le tensioactif amphotère ou zwitterionique est choisi parmi les bétaïnes, et notamment les alkylbétaïnes, les alkylamphoacétates tels que le sodium cocamphoacetate, et leurs mélanges.

La composition selon l'invention peut contenir, outre les tensioactifs moussants anioniques ou amphotères, un ou plusieurs tensioactifs moussants additionnels choisis parmi les tensioactifs non ioniques, les tensioactifs cationiques et leurs mélanges.

### Tensioactif moussant non ionique

Les tensioactifs moussants non ioniques de la composition de l'invention peuvent être choisis notamment parmi les alkyl polyglucosides (APG), les esters de glycérol oxyalkylénés, les esters de sucre oxyalkylénés, et leurs mélanges. Ce sont de manière préférée des APG.

Comme alkylpolyglucosides, on utilise de préférence ceux contenant un groupe alkyle comportant de 6 à 30 atomes de carbone et de préférence de 8 à 16 atomes de carbone, et contenant un groupe glucoside comprenant de préférence 1,2 à 3 unités de glucoside. Les alkylpolyglucosides peuvent être choisis par exemple parmi le decylglucoside (Alkyl-C9/C11-polyglucoside (1.4)) comme le produit commercialisé sous la dénomination Mydol 10 ® par la société Kao Chemicals ou le produit commercialisé sous la dénomination Plantacare 2000 UP® par la société Cognis ; le caprylyl/capryl glucoside comme le produit commercialisé sous la dénomination Plantacare KE 3711® par la société Cognis ; le laurylglucoside comme le produit commercialisé sous la dénomination Plantacare 1200 UP® par la société Cognis ; le cocoglucoside comme le produit commercialisé sous la dénomination Plantacare 818 UP® par la société Cognis ; le caprylylglucoside comme le produit commercialisé sous la dénomination Plantacare 810 UP® par la société Cognis ; et leurs mélanges.

Les esters de glycérol oxyalkylénés sont notamment les dérivés polyoxyéthylénés des esters de glycéryle et d'acide gras et de leurs dérivés hydrogénés. Ces esters de glycérol oxyalkylénés peuvent être choisis par exemple parmi les esters de glycéryle et d'acides gras hydrogénés et oxyéthylénés tel que le PEG-200 hydrogenated glyceryl palmate commercialisé sous la dénomination Rewoderm LI-S 80 par la société Goldschmidt ; les cocoates de glycéryle oxyéthylénés comme le PEG-7 glyceryl cocoate commercialisé sous la dénomination Tegosoft GC par la société Goldschmidt, et le PEG-30 glyceryl cocoate commercialisé sous la dénomination Rewoderm LI-63 par la société Goldschmidt ; et leurs mélanges.

Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioleate commercialisé sous la dénomination Glucamate DOE 120 par la société Amerchol.

Selon un mode préféré de réalisation de l'invention, le tensioactif non ionique est un alkylpolyglucoside qui peut être choisi notamment parmi le decylglucoside, le caprylyl/capryl glucoside, le laurylglucoside, le cocoglucoside, le caprylylglucoside, et leurs mélanges.

### Tensioactif moussant cationiques

Selon un mode de réalisation, la composition selon l'invention peut comprendre au moins un tensioactif cationique, en particulier dans le cas où elle comprend un tensioactif moussant amphotère. Les tensioactifs cationiques utilisables selon la présente invention sont notamment les sels d'amines grasses primaires, secondaires ou tertiaires, éventuellement polyoxyalkylénées ; les sels d'ammonium quaternaire ; les dérivés d'imidazoline ; les oxydes d'amines à caractère cationique, et/ou l'un de leurs mélanges.

Les sels d'ammonium quaternaires sont par exemple :
- ceux qui présentent la formule générale (IV) suivante : 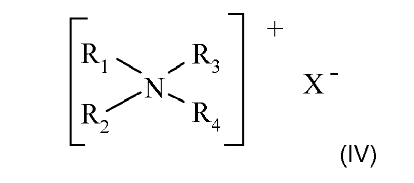 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates, De préférence R₁ et R₂ désigne un alkyle en C₁-C₄, ou un hydroxyalkyle en C₁-C₄.
- les sels d'ammonium quaternaire de l'imidazolinium, comme par exemple celui de formule (V) suivante : 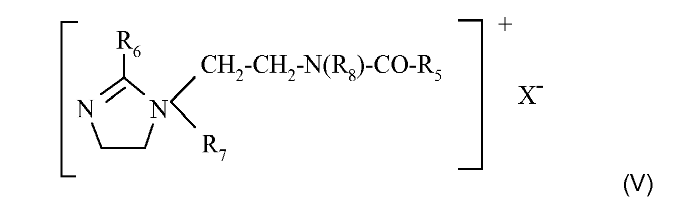 dans laquelle R₅ représente un radical alcényle ou alkyle comportant de 8 à 30 atomes de carbone par exemple dérivés des acides gras de coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X⁻ est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.
- les sels de diammonium quaternaire de formule (VI) : 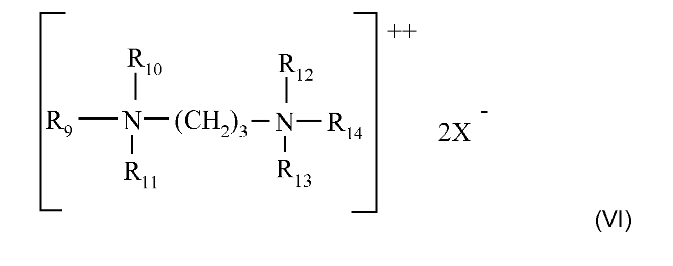 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthyl-sulfates.
- les sels d'ammonium quaternaire contenant au moins une fonction ester par exemple ceux de formule (VII) suivante : 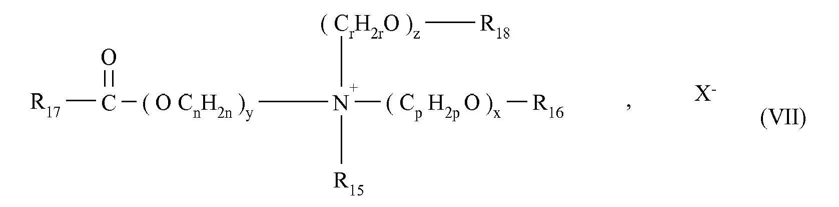 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 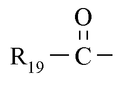
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 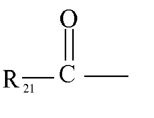
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;

Sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 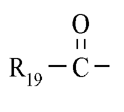
   - les radicaux méthyle, éthyle ou hydrocarbonés en C₁₄-C₂₂
   - l'atome d'hydrogène ;
- R₁₈ est choisi parmi :
   - le radical 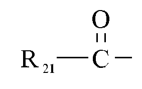
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.

Avantageusement, les radicaux hydrocarbonés sont linéaires.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyldiméthylstéaryl ammonium ou encore, d'autre part, le chlorure de palmitylamidopropyltriméthylammonium ou le chlorure de stéaramidopropyldiméthyl (myristyl acétate) ammonium commercialisé sous la dénomination "CERAPHYL 70" par la société VAN DYK.

On peut citer par exemple les composés de formule (V) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 et REWOQUAT W75 par la société DEGUSSA.

On peut aussi utiliser les sels d'ammonium contenant au moins une fonction ester décrits dans les brevets US-A-4874554 et US-A-4137180.

Des sels de diammonium quaternaire de formule (VI) convenant à l'invention comprennent notamment le dichlorure de propanesuif diammonium.

La quantité (en matière active) de tensioactif(s) moussant(s) additionnels dans la composition selon l'invention peut aller par exemple de 0,1 à 10% en poids, de préférence de 0,2 à 7% en poids et mieux de 0,5% à 5% en poids par rapport au poids total de la composition.

La composition peut comprendre, outre les tensioactifs anioniques et amphotères et les tensioactifs additionnels, un sel d'ammonium quaternaire polymérique (distinct des tensioactifs précédents) et/ou une gomme de galactomannane cationique, de préférence une gomme de guar cationique.

Ces composés permettent d'augmenter la quantité de mousse et d'obtenir une sensation de douceur de confort sur la peau (maintien de l'hydratation).

Les sels d'ammonium quaternaire polymériques sont des polymères cationiques ou amphotères contenant au moins un atome d'azote quaternisé. Comme sels d'ammonium quaternaire polymériques, on peut citer notamment les Polyquaternium (nom CTFA), qui apportent douceur et onctuosité à la crème moussante. Ces polymères peuvent être choisis de préférence parmi les polymères suivants :
● Polyquaternium 5 tel que le produit MERQUAT 5 commercialisé par la société CALGON ;
● Polyquaternium 6 tel que le produit SALCARE SC 30 commercialisé par la société CIBA, et le produit MERQUAT 100 commercialisé par la société CALGON ;
● Polyquaternium 7 tel que les produits MEROUATS, MEROUAT 2200 et MEROUAT 550 commercialisés par la société CALGON, et le produit SALCARE SC 10 commercialisé par la société CIBA ;
● Polyquaternium 10 tel que le produit Polymer JR400 commercialisé par la société AMERCHOL;
● Polyquaternium 11 tel que les produits GAFQUAT 755, GAFQUAT 755N et GAFOUAT 734 commercialisés par la société ISP ;
● Polyquaternium 15 tel que le produit ROHAGIT KF 720 F commercialisé par la société ROHM ;
● Polyquaternium 16 tel que les produits LUVIQUAT FC905, LUVIQUAT FC370, LUVIQUAT HM552 et LUVIQUAT FC550 commercialisés par la société BASF ;
● Polyquaternium 22 tel que le produit MEROUAT 280 commercialisé par la société CALGON ;
● Polyquaternium 28 tel que le produit STYLEZE CC10 commercialisé par la société ISP ;
● Polyquaternium 39 tel que le produit MERQUAT PLUS 3330 commercialisé par la société CALGON ;
● Polyquaternium 44 tel que le produit LUVIQUAT CARE commercialisé par la société BASF;
● Polyquaternium 46 tel que le produit LUVIQUAT HOLD commercialisé par la société BASF;
● Polyquaternium 47 tel que le produit MERQUAT 2001 commercialisé par la société CALGON.

De manière préférée, les sels d'ammonium quaternaire sont choisis parmi le Polyquaternium-7, le Polyquaternium-10, le Polyquaternium-39, le Polyquaternium-47, et leurs mélanges.

Les gommes de galactomannane cationiques ont de préférence une densité de charge cationique inférieure ou égale à 1,5 meq/g et plus particulièrement comprise entre 0,1 et 1 meq/g.
De manière générale, au sens de la présente invention, on entend par "gomme de galactomannane cationique" toute gomme de galactomannane contenant des groupements cationiques et/ou des groupements ionisables en groupements cationiques.
Les groupements cationiques préférés sont choisis parmi ceux comportant des groupements amine primaires, secondaires, tertiaires et/ou quaternaires.
Les gommes de galactomannane cationiques utilisées ont généralement une masse moléculaire moyenne en poids comprise entre 500 et 5.106 environ, et de préférence comprise entre 103 et 3.106 environ.
Les gommes de galactomannane cationiques utilisables selon la présente invention sont par exemple des gommes comportant des groupements cationiques trialkyl(C1-C4)ammonium. De préférence, 2 à 30% en nombre des fonctions hydroxyle de ces gommes porte des groupements cationiques trialkylammonium.
Parmi ces groupements trialkylammonium, on peut tout particulièrement citer les groupements triméthylammonium et triéthylammonium.
Encore plus préférentiellement, ces groupements représentent de 5 à 20% en poids du poids total de la gomme de galactomannane modifiée.
Selon l'invention, on utilise de préférence une gomme de guar comportant des groupements hydroxypropyl triméthylammonium, c'est à dire une gomme de guar modifiée par exemple par du chlorure de 2,3-époxypropyl triméthylammonium.
Ces gommes de galactomannane en particulier de guar modifiées par des groupements cationiques sont des produits déjà connus en eux-mêmes et sont par exemple décrits dans les brevets US 3 589 578 et US 4 031 307. De tels produits sont par ailleurs vendus notamment sous les dénominations commerciales de JAGUAR C13S, JAGUAR C15, JAGUAR C17 et JAGUAR C162 par la société MEYHALL.

Les sels d'ammonium quaternaire polymériques et/ou les gommes de galactomannane cationiques peuvent être en une quantité (en matière active) allant par exemple de 0,01 à 5 % en poids et mieux de 0,05 à 1 % en poids par rapport au poids total de la composition.

Selon un mode de réalisation, la composition selon l'invention comprend une phase aqueuse contenant de l'eau et éventuellement un ou plusieurs solvants organiques hydrosolubles. Dans ce milieu aqueux, la quantité d'eau est de préférence d'au moins 20 % en poids; elle va de préférence de 20 à 95 % en poids, mieux de 30 à 90 % en poids et encore mieux de 40 à 85 % en poids par rapport au poids total de la composition.

Les solvants hydrosolubles peuvent être choisis parmi les alcool(s) inférieur(s) hydrosoluble(s). On entend par alcool inférieur, un alcool comportant de 1 à 8 atomes de carbone. Comme alcools inférieurs, on peut citer par exemple l'éthanol, l'isopropanol, le butanol et leurs mélanges. Lorsqu'ils sont présents dans la composition de l'invention, le ou les alcool(s) inférieur(s) hydrosoluble(s) peuvent être en une quantité allant de 0,01 à 40 % en poids.

La phase aqueuse peut également comprendre des polyols (ou alcools polyhydriques) comme par exemple la glycérine; les glycols comme le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges. La quantité de polyols va généralement de 0,1 à 60 % en poids et mieux de 0,5 à 50 % en poids par rapport au poids total de la phase aqueuse.

Selon un mode de réalisation, la composition selon l'invention comprend moins de 5% en poids d'eau, de préférence moins de 3% et mieux moins de 2%, elle peut être totalement exempte d'eau (composition anhydre). Elle peut toutefois comprendre des solvants hydrosolubles tels que les alcools inférieurs ou les polyols cités plus haut.

### Huiles

La composition selon l'invention peut comprendre une huile. Comme huiles utilisables dans la composition de l'invention, on peut citer par exemple :
- les huiles essentielles
- les huiles hydrocarbonées d'origine animale, telles que le perhydrosqualène ;
- les huiles hydrocarbonées d'origine végétale, telles que les triglycérides liquides d'acides gras comportant de 4 à 30 atomes de carbone comme les triglycérides des acides heptanoïque ou octanoïque ou encore, par exemple, les huiles de tournesol, de jojoba, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de beurre de karité;

- les esters et les éthers de synthèse, notamment d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

L'huile est peut être présente en teneur allant de à 0.5% à 15% en poids par rapport au poids total de la composition, de préférence inférieure ou égale à 1% à 12% en poids.

Les compositions de l'invention peuvent contenir aussi des adjuvants habituellement utilisés dans le domaine cosmétique. Comme adjuvants, on peut citer par exemple les parfums, les conservateurs, les séquestrants (EDTA), les pigments, les charges notamment exfoliantes, les colorants solubles, les filtres solaires, les actifs cosmétiques ou dermatologiques tels que les hydratants comme l'acide hyaluronique ; les céramides ; les vitamines hydrosolubles ou liposolubles comme la vitamine C et ses dérivés tels que la vitamine CG ; les antiseptiques ; les antiséborrhéïques ; les antimicrobiens tels que le peroxyde de benzoyle, l'acide salicylique, le triclosan, l'acide azélaïque ; les azurants optiques. Les quantités de ces différents adjuvants sont celles classiquement utilisées dans le domaine considéré, et par exemple de 0,01 à 5 % du poids total de la composition. Ces adjuvants ainsi que leurs concentrations doivent être tels qu'ils ne modifient pas la propriété recherchée pour la composition de l'invention.

Comme exfoliants, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Ces particules peuvent être présentes en une quantité allant par exemple de 0,5 à 40 % en poids, de préférence de 1 à 20 % en poids et mieux de 1 à 10 % en poids par rapport au poids total de la composition. Quand la composition contient des particules exfoliantes, elle peut constituer notamment une composition de gommage de la peau du visage ou du corps.

Les compositions de l'invention peuvent contenir aussi des polymères, notamment anioniques et non ioniques.

Comme polymères anioniques, on peut citer notamment ceux comportant au moins une chaîne hydrophobe, et en particulier ceux dérivés d'acide acrylique ou méthacrylique, comme le copolymère acrylates/steareth-20 methacrylate commercialisé sous la dénomination Aculyn 22 par la société Rohm & Haas (nom CTFA : Acrylates/Steareth-30 Methacrylate copolymer) ; le terpolymère acide (méth)acrylique / acrylate d'éthyle / méthacrylate de béhényle oxyéthyléné (25 OE), en émulsion aqueuse commercialisé sous la dénomination Aculyn 28 par la société Rohm & Haas ; le copolymère acide acrylique/itaconate de mono-cétyle oxyéthyléné (20 OE), en dispersion aqueuse à 30 % commercialisé sous la dénomination Structure 3001 par la société National Starch ; le copolymère acide acrylique/itaconate de mono-stéaryle oxyéthyléné (20 OE) en dispersion aqueuse à 30 % commercialisé sous la dénomination Structure 2001 par la société National Starch ; le copolymère acrylates/acrylate modifié par des alcools en C12-C24 polyoxyéthylénés (25 OE), sous forme de latex à 30-32 % de copolymère, commercialisé sous la dénomination Synthalen W2000 par la société 3V SA, ainsi que les copolymères vendus sous les noms PEMULEN ou CARBOPOL par la Société Noveon, comme le copolymère acrylate/C₁₀-C₃₀-alkylacrylate tel que les produits PEMULEN TR1 ou CARBOPOL 1382 (nom CTFA : Acrylates/C10-30 Alkyl acrylate Crosspolymer).

La composition peut contenir aussi des polymères comportant au moins un monomère à groupement sulfonique, et notamment les polymères et copolymères d'acide 2-acrylamido-2-méthylpropane sulfonique (AMPS) tels que :
- l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide) ;
- les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer / Isohexadecane / Polysorbate 80),
- les copolymères d'acide(méth)acrylique ou de (méth)acrylate et d'acide 2-acrylamido-2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom CTFA : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom C.T.F.A. : Sodium Acrylate / Sodium acryloydimethyl-taurate copolymer Isohexadecane / Polysorbate 80),
- les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant,
- les polymères d'AMPS modifiés hydrophobes comme notamment le copolymère d'AMPS et de methacrylate d'alcool en C12-C14 éthoxylé (copolymère non réticulé obtenu à partir de Genapol LA-070 et d'AMPS) (nom CTFA: Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le trimethylolpropane triacrylate, obtenu à partir de Genapol T-250 et d'AMPS) (nom CTFA : Ammonium Acryloyldimethyltaurate / Steareth-25 Methacrylate Crosspolymer) commercialisé sous la dénomination ARISTOFLEX HMS par la société Clariant.

Ces polymères peuvent être en une quantité (en matière active) allant par exemple de 0,05 à 10 % en poids et mieux de 0,1 à 5 % en poids par rapport au poids total de la composition.

Les compositions selon l'invention peuvent être utilisées sur toutes les matières kératiniques telles que la peau, le cuir chevelu, les cheveux, les cils, les sourcils, les ongles ou les muqueuses, notamment comme produit d'hygiène, par exemple comme produit de nettoyage de la peau, des muqueuses et/ou des cheveux, en particulier comme produit de nettoyage et/ou de démaquillage de la peau (du visage et/ou du corps), comme produit de douche (produit deux-en-un), comme shampoing et après-shampoing, comme produit de rasage, comme masque à rincer, et comme produit exfoliant (appelé aussi desquamant ou désincrustant) aussi bien pour le visage que pour le corps ou pour les mains, après addition de particules exfoliantes.

L'invention a encore pour objet l'utilisation cosmétique de la composition telle que définie ci-dessus, comme produit de nettoyage et/ou de démaquillage de la peau, comme produit de douche, comme shampoing, comme produit après-shampoing, comme produit de rasage, comme masque à rincer, comme produit exfoliant.

Un autre objet de l'invention est un procédé de nettoyage d'une matière kératinique, telle que la peau, du cuir chevelu, des cheveux, des cils, des sourcils, des ongles ou des muqueuses, caractérisé par le fait qu'on applique sur la matière kératinique, une composition telle que définie ci-dessus, et qu'on la rince.

La matière kératinique est de préférence la peau.

Les compositions selon l'invention peuvent être par exemple utilisées de la manière suivante :
1) quand les compositions selon l'invention constituent des produits de nettoyage doux pour le visage, elles peuvent être utilisées de la manière suivante :
   - on fait mousser le produit dans les mains avec de l'eau
   - on applique la mousse sur le visage
   - on nettoie le visage
   - on rince à l'eau
2) quand les compositions selon l'invention constituent des produits démaquillants, elles peuvent être utilisées comme indiqué ci-dessus mais elles peuvent aussi être appliquées à sec sur le visage, puis massées jusqu'à obtention d'un démaquillage satisfaisant, et rincées à l'eau, ou bien elles peuvent être appliquées au moyen d'un coton.
3) on peut les utiliser comme produit de douche deux-en-un, comme shampooing et/ou après shampooing, comme masque à rincer ou comme produit de rasage, de la manière habituelle d'utilisation de ces produits.

Les exemples qui suivent servent à illustrer l'invention sans toutefois présenter un caractère limitatif. Les quantités indiquées sont en % en poids sauf mention contraire, et elles correspondent sauf mention contraire à la quantité de matière première et non à la quantité de matière active. Les noms des composés utilisés sont indiqués en nom CTFA, en nom chimique ou en nom commercial.

### Exemples

Dans les exemples qui suivent, les performances de mousse (qualités de mousse) sont évaluées selon le protocole suivant :
Avant toute utilisation du produit, les mains sont lavées au savon de Marseille puis convenablement rincées et séchées. Puis le protocole suivi est le suivant :
   1- mouiller les mains en les passant sous l'eau courante, les secouer trois fois pour les enlever l'excès d'eau,
   2- placer 1 g de produit dans le creux de l'une des mains,
   3- travailler le produit entre les deux paumes pendant 10 secondes,
   4- ajouter 2 ml d'eau et travailler le produit à nouveau pendant 10 secondes,
   5- rincer les mains sous l'eau ,
   6- les essuyer.

Les critères suivants sont évalués :
- volume de mousse : la note attribuée est d'autant plus élevée que le volume est grand.
- taille des bulles composant la mousse : la note attribuée est d'autant plus élevée que les bulles sont grosses.
- densité : consistance, tenue de la mousse ; la note attribuée est d'autant plus élevée que la densité est grande.

Ils sont notés sur une échelle de 0 à 10. On considère que, pour un critère donné, il y a une différence de note entre deux produits lorsque la différence entre 2 notes est supérieure ou égale à 0,5.

Le panel d'évaluation est constitué de 3 experts entraînés. La moyenne des 3 notes permet de comparer les compositions selon chacun des critères.

### Exemples 1 à 3

On prépare les compositions suivantes :

| | **Exemple 1 (invention)** | **Exemple 1' (comparatif)** | **Exemple 2 (invention)** | **Exemple 2' (comparatif)** | **Exemple 3 (invention)** | **Exemple 3' (comparatif)** |
|---|---|---|---|---|---|---|
| Lauryl Ether Sulfate (70% MA) (1) | 18.6% | 18.6% | - | - | - | - |
| Laureth-5 carboxylic acid (90% | - | - | 14.44% | 14.44% | - | - |
| MA) (2) | | | | | | |
| Cocobetaine (30% MA) (3) | - | - | - | - | 43.33% | 43.33% |
| Sel de sodium de l'acide 3- | 6.67% | - | 6.67% | - | 6.67% | - |
| hydroxy-2-pentyl-cyclopentane | | | | | | |
| acétique à 30 % dans un mélange | | | | | | |
| eau/dipropylène glycol (70/30). | | | | | | |
| Eau | 74.73% | 81.4% | 69.75% | 75.56% | 50% | 56.67% |
| Soude 10% dans l'eau | - | - | 9.14% | 10% | - | - |
| **Aspect** | Solution limpide transparente | Solution limpide transparente | Solution limpide transparente | Solution limpide transparente | Solution limpide transparente | Solution limpide transparente |
| **pH** | 7.2 | 8 | 6.7 | 6.8 | 6.9 | 7.5 |
| **Notes:** | | | | | | |
| Avant ajout d'eau : | | | | | | |
| Volume de mousse | **3.2** | 2.7 | **3.5** | 2.2 | **4.5** | 3.5 |
| Taille de bulles | 3.8 | 3.7 | 3.8 | 2.3 | 6.5 | 6.3 |
| Densité de la mousse | **7.5** | 6.2 | **7** | 5.3 | **7.3** | 6.8 |
| Après ajout d'eau : | | | | | | |
| Volume de mousse | **6.7** | 5.7 | **6.3** | 4.7 | **7.7** | 6.5 |
| Taille de bulles | **5** | 5.5 | **4** | 4.2 | **6.7** | 6.3 |
| Densité de la mousse | **6.8** | 5.3 | **6.7** | 5.5 | **9.7** | 9 |

| | | | | | | |
|---|---|---|---|---|---|---|
| (1) TEXAPON AOS 225 UP (COGNIS) (2) AKYPO RLM 45 CA (KAO) (3) GENAGEN KB (CLARIANT) | | | | | | |

Mode opératoire : les mélanges de tensioactifs sont réalisés à température ambiante au barreau aimanté. Le sel d'acide 3-hydroxy-2-pentyl-cyclopentane acétique si présent est ajouté à froid à la solution de tensioactifs.

Ces exemples montrent que l'addition du dérivé d'acide jasmonique augmente le volume et la densité de mousse, à taille de bulle identique, dans des bases tensioactives anioniques ou amphotères.

### Exemples 4 et 4' : Crèmes nettoyantes moussantes

| | Phase | **Exemple 4 (invention)** | **Exemple 4' (comparatif)** |
|---|---|---|---|
| Eau | A1 | 8.42% | 8.42% |
| Carbomer (1) | A2 | 0.25% | 0.25% |
| Glycerine | A3 | 39.3% | 39.3% |
| Guar hydroxypropyl trimonium chloride (30% MA) (2) | A4 | 0.25% | 0.25% |
| Sodium cocoyl glycinate (52% MA) (3) | B1 | 43.33% | 43.33% |
| Glycol distearate | B2 | 1.8% | 1.8% |
| Stearic acid | B3 | 1.1 % | 1.1 % |
| Polyquaternium-39 (10% MA) (4) | C | 2.5% | 2.5% |
| Acide citrique (20%) | D | 2.55% | 2.55% |
| Parfum | E | 0.5% | 0.5% |
| Sel de sodium de l'acide 3-hydroxy-2-pentyl-cyclopentane acétique à 30 % dans un mélange eau/dipropylène glycol (70/30). | F | 6.67% | - |
| **Aspect** | | Crème blanche | Crème blanche |
| **pH** | | 7.0 | 7.0 |
| **Notes sensorielles** | | | |
| Avant ajout d'eau : | | | |
| Volume de mousse | | 5.3 | **6.5** |
| Taille de bulles | | 2.3 | 2.5 |
| Densité de la mousse | | 7 | **8** |
| Après ajout d'eau : | | | |
| Volume de mousse | | 7.5 | **8.5** |
| Taille de bulles | | 2.5 | 2.5 |
| Densité de la mousse | | 7.5 | **8** |

| | | | |
|---|---|---|---|
| (1) CARBOPOL ULTREZ 10 (LUBRIZOL) (2) JAGUAR C 162 (RHODIA) (3) AMILITE GCS-12K (AJINOMOTO) (4) MERQUAT PLUS 3330 (NALCO) | | | |

### Mode opératoire :

1- Préparer la phase A = A1 + A2 + A3 + A4 sous défloculeuse à température ambiante.
2- Chauffer la phase B = B1 + B2 + B3 à 70-75°C au bain marie.
3- Introduire progressivement la phase aqueuse A dans la phase grasse B chaude, homogénéiser 10 min à 50-55°C
4- Incorporer C, D, E et F
5- Refroidir sous défloculeuse

Ces exemples montrent que la présence du sel d'acide 3-hydroxy-2-pentyl-cyclopentane acétique améliore significativement la qualité de mousse d'un composition comprenant un tensioactif composition comprenant un tensioactif anionique.
La composition de l'exemple 4 selon l'invention donne une mousse abondante et dense, elle se rince bien et elle est bien tolérée par la peau. Elle peut être avantageusement utilisée pour le nettoyage de la peau du visage.

### Exemples 5 et 5' : Crèmes savon liquides moussantes

| | **Exemple 5** | **Exemple 5'** |
|---|---|---|
| Lauryl Ether sulfate de sodium (70% MA) (1) | 30.33% | 30.33% |
| Alkyl Polyglucoside (52% MA) (2) | 7.5% | 7.5% |
| Eau | 62.17% | 55.5% |
| Sel de sodium de l'acide 3-hydroxy-2-pentyl-cyclopentane acétique à 30 % dans un mélange eau/dipropylène glycol (70/30). | | 6.67% |
| Acide citrique (10% MA) | Qsp pH | Qsp pH |
| **Aspect** | Solution transparente | Solution transparente |
| **pH** | 7.1 | 7.2 |
| **Notes sensorielles** | | |
| Avant ajout d'eau : | | |
| Volume de mousse | 4.5 | **7** |
| Taille de bulles | 5.3 | 4.3 |
| Densité de la mousse | 5.5 | **7.3** |
| Après ajout d'eau : | | |
| Volume de mousse | 6 | **9.5** |
| Taille de bulles | 6.5 | 5 |
| Densité de la mousse | 2.5 | **7** |

| | | |
|---|---|---|
| (1) TEXAPON AOS 225 UP (COGNIS) (2) PLANTACARE 818 UP (COGNIS) | | |

La présence du sel d'acide 3-hydroxy-2-pentyl-cyclopentane acétique améliore significativement la qualité de mousse de la composition selon l'invention.

## Revendications

1. Composition de nettoyage comprenant (1) au moins un tensioactif moussant anionique ou amphotère et (2) au moins un composé dérivé d'acide jasmonique choisi parmi ceux répondant à la formule (I) suivante : 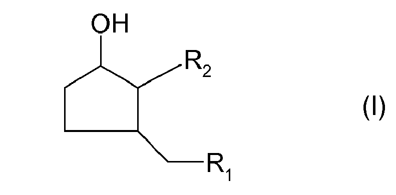 dans laquelle :
- R1 représente un radical COOR3, R3 désignant un atome d'hydrogène ou un radical alkyle en C1-C4, éventuellement substitué par un ou plusieurs groupes hydroxyle ;
- R2 représente un radical hydrocarboné, saturé ou insaturé, linéaire ayant de 1 à 18 atomes de carbones, ou ramifié ou cyclique ayant de 3 à 18 atomes de carbone ;
ainsi que leurs isomères optiques, et sels correspondants.

2. Composition selon la revendication 1, **caractérisée en ce que**, dans le composé de formule (I), R₁ désigne un radical choisi parmi ―COOH, -COOMe, -COO-, CH2-CH3, - COO—CH2-CH(OH)-CH2OH, -COOCH2-CH2-CH20H, -COOCH2-CH(OH)-CH3.

3. Composition selon la revendication 1 ou 2, **caractérisée en ce que**, dans le composé de formule (I), R1 désigne un radical ―COOH.

4. Composition selon l'une quelconque des revendications précédentes, **caractérisée en ce que**, dans le composé de formule (I), R₂ désigne un radical hydrocarboné, linéaire, saturé ou insaturé, et de préférence ayant de 2 à 7 atomes de carbone.

5. Composition selon l'une quelconque des revendications précédentes, **caractérisée en ce que** R2 est un radical pentyl, pentenyl, hexyle, heptyle.

6. Composition selon l'une quelconque des revendications précédentes, **caractérisée en ce que** le composé de formule (I) est choisi parmi l'acide 3-hydroxy-2-[(2Z)-2-pentenyl]-cyclopentane acétique ou l'acide 3-hydroxy 2-pentyl cyclopentane acétique.

7. Composition selon l'une quelconque des revendications précédentes, **caractérisée en ce que** les sels des composés de formule (I) sont choisis parmi les sels de métal alcalin, les sels de métal alcalino-terreux, les sels métalliques, les sels d'ammonium de formule NH₄⁺ ; les sels d'ammonium quaternaires ; les sels d'amines organiques, les sels de lysine, d'arginine.

8. Composition selon l'une quelconque des revendications précédentes, **caractérisée en ce que** le composé de formule (I) est présent en une quantité allant de 0,1 à 15 % en poids, de préférence de 0,2 à 10% en poids et mieux de 0,5 à 10% en poids par rapport au poids total de la composition.

9. Composition selon l'une quelconque des revendications précédentes, **caractérisée en ce que** les tensioactifs moussants anioniques et/ou amphotères sont présents en une quantité (en matière active) allant de 0,1 à 40 % en poids, de préférence de 0,5 à 30 % en poids, mieux de 1 à 25% et encore mieux de 3 à 20% en poids par rapport au poids total de la composition.

10. Composition selon l'une quelconque des revendications précédentes, **caractérisée en ce que** le tensioactif anionique est choisi parmi les dérivés anioniques de protéines d'origine végétale ou de protéines de soie, les phosphates et alkylphosphates, les carboxylates, les sulfosuccinates, les dérivés des aminoacides, les alkyl sulfates, les alkyl éther sulfates, les sulfonates, les iséthionates, les taurates, les alkyl sulfoacétates, les polypeptides, les dérivés anioniques d'alkyl polyglucoside, les savons (sels d'acides gras) et leurs mélanges.

11. Composition selon l'une quelconque des revendications précédentes, **caractérisée en ce que** le tensioactif anionique est choisi parmi les dérivés des aminoacides, les alkyl sulfates, les alkyl éther sulfates, les iséthionates, les dérivés de aminoacides, et leurs mélanges.

12. Composition selon la revendication précédente, **caractérisée en ce que** le tensioactif amphotère ou zwitterionique est choisi parmi les bétaïnes, les N-alkylamidobétaïnes et leurs dérivés, les sultaïnes, les alkyl polyaminocarboxylates, les alkylamphoacétates et leurs mélanges.

13. Composition selon la revendication précédente, **caractérisée en ce que** le tensioactif amphotère ou zwitterionique est choisi parmi les bétaïnes, et notamment les alkylbétaïnes, les alkylamphoacétates et leurs mélanges.

14. Composition selon l'une quelconque des revendications précédentes, **caractérisée en ce qu'**elle comprend en outre au moins un sel d'ammonium quaternaire polymérique (distinct des tensioactifs précédents) et/ou au moins une gomme de galactomannane cationique, de préférence une gomme de guar cationique.

15. Procédé de nettoyage d'une matière kératinique, **caractérisé en ce qu'**on applique sur la matière kératinique, une composition selon l'une quelconque des revendications 1 à 14, et qu'on la rince.
